# EUROPEAN PATENT APPLICATION

(11) **EP 2 345 633 A1**
(43) Date of publication of application: **20.07.2011**
(21) Application number: 09819272.7
(22) Date of filing: 09.10.2009
(51) Int. Cl.: C07C 69/757, C07C 67/52, C07C 211/27, C07C 211/29, C07C 211/30, C07C 227/32, C07C 229/28, C07C 231/20, C07C 235/82, C07C 269/04, C07C 271/24

(54) **OPTICALLY ACTIVE VINYL-CYCLOPROPANE CARBOXYLIC ACID DERIVATIVE AND OPTICALLY ACTIVE VINYL-CYCLOPROPANE AMINO ACID DERIVATIVE MANUFACTURING METHOD**

(30) Priority: 10.10.2008 JP 2008263612
(71) Applicant: Kaneka Corporation, Osaka-shi, Osaka 530-8288 (JP)
(72) Inventor: TANAKA, Tatsuyoshi, Takasago-shi Hyogo 676-8688 (JP); OKURO, Kazumi, Osaka-shi Osaka 530-8288 (JP)
(74) Representative: HOFFMANN EITLE
(86) International application number: PCT/JP2009/067639
(87) International publication number: WO 2010/041739

(57) **Abstract**

The objective of the present invention is to provide a method for obtaining an optically active vinylcyclopropanecarboxylic acid derivative with high yield and high optical purity using a safe material available at low cost. In addition, the objective of the present invention is to provide a method for safely-obtaining an optically active vinylcyclopropaneamino acid with high optical purity at low cost. The problems can be solved by a method for obtaining an optically active vinylcyclopropanecarboxylic acid derivative, which method contains the step of reacting a racemic vinylcyclopropanecarboxylic acid derivative with an optically active amine compound, to obtain a diastereomer salt of optically active vinylcyclopropanecarboxylic acid derivative - amine compound. In addition, it is possible to obtain a vinylcyclopropaneamino acid by deriving the vinylcyclopropaneamino acid from thus obtained diastereomer salt of optically active vinylcyclopropanecarboxylic acid derivative - amine compound.

## Description

### TECHNICAL FIELD

The present invention relates to a method for producing an optically active vinylcyclopropanecarboxylic acid derivative and an optically active vinylcyclopropaneamino acid derivative which are especially useful as an intermediate of a drug against hepatitis C.

### BACKGROUND ART

As the method for producing an optically active vinylcyclopropanecarboxylic acid derivative, the following methods have been known:
1) the method in which a racemic vinylcyclopropanemalonic acid diester is synthesized by reacting malonic acid diester with 1,4-dibromo-2-butene, and then the racemic substance is resolved by lipase (Non-patent Document 1);
2) the method in which racemic vinylcyclopropanedicarboxylic acid is reacted with an optically active amine compound to be diastereomer salt for optical resolution (Patent Document 1).

However, in the method 1), the selectivity of resolution by enzyme is low. In addition, a large amount of enzyme is required, since the reactivity thereof is also low. Furthermore an enzyme derived from pig is needed; therefore the method 1) is not preferable as an industrial production method. In the method 2), there are problems that a large amount of organic solvent is needed during extraction procedure and the productivity is low, since the solubility of vinylcyclopropanecarboxylic acid to water is high. In addition, the method 2) has a problem that the resolution efficiency is as low as 78%ee or less when racemic vinylcyclopropanecarboxylic acid is optically resolved and the subsequent purification procedure is burdensome for meeting the standard for pharmaceutical intermediate which generally requires high optical purity.

In addition, amino acid derivative or amino alcohol derivative is used as an amine resolving agent in the method 2), and the synthesis thereof is difficult and inefficient. Furthermore, the method 2) has a problem that the cost for producing the resolving agent becomes very high depending on the desired configuration of vinylcyclopropanecarboxylic acid, since though the amino acid compound as a material of such a resolving agent which has the natural type configuration is relatively easily-obtainable, the amino acid compound which has the opposite configuration is difficult to be obtained and expensive.

In addition, the method using Curtius rearrangement has been known as a method in which a vinylcyclopropanecarboxylic acid derivative is used and the derivative is transformed into a vinylcyclopropaneamino acid derivative. However, the method is not suitable for industrial production, since an azide compound with explosion hazard has to be used in the reaction and it is difficult to control the amount of nitrogen generated during the reaction (Patent Document 1 and Non-patent Document 1).

### PRIOR ART

### Patent Document

Patent Document 1 : WO2007/088571

### Non-patent Document

Non-patent Document 1 : Synthetic Communications, 1994, 24, 2873

### DISCLOSURE OF THE INVENTION

### PROBLEMS TO BE SOLVED BY THE INVENTION

The objective of the present invention is to provide a method for safely obtaining an optically active vinylcyclopropanecarboxylic acid derivative with high optical purity at low cost, and is to safely-provide a vinylcyclopropaneamino acid with high optical purity at low cost.

### MEANS FOR SOLVING THE PROBLEMS

The present inventors studied very hard for solving the problems. As a result, the present inventors found the method by which an optically active vinylcyclopropanecarboxylic acid derivative can be effectively synthesized by efficiently-carriying out resolution from the racemic vinylcyclopropanecarboxylic acid derivative which can be easily synthesized.

The present invention relates to a method for producing an optically active vinylcyclopropanecarboxylic acid derivative, comprising the step of reacting a racemic vinylcyclopropanecarboxylic acid derivative represented by the general formula (1): 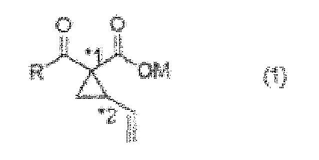 wherein, R is NH₂, a substituted or unsubstituted alkoxy group having 1 to 4 carbon atoms, a substituted or unsubstituted aryloxy group having 6 to 10 carbon atoms, or a substituted or unsubstituted aralkyloxy group having 7 to 11 carbon atoms; M is a hydrogen atom or a metal atom; *1 and *2 indicate asymmetric carbon atoms,
with an optically active amine compound, to obtain a diastereomer salt of optically active vinylcyclopropanecarboxylic acid derivative and amine compound.

The present invention is related to a diastereomer salt of optically active vinylcyclopropanecarboxylic acid derivative - amine compound, represented by the general formula (3): 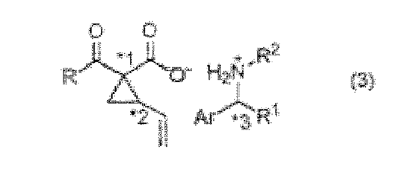 wherein R¹ is an alkyl group having 1 to 3 carbon atoms; R² is a substituted or unsubstituted aralkyl group having 7 to 11 carbon atoms, or a hydrogen atom; Ar is a substituted or unsubstituted aryl group having 6 to 10 carbon atoms; R, *1 and *2 are the same as the above; *3 indicates an asymmetric carbon atom.

In addition, the present inventors hardly studied the method for transforming a vinylcyclopropanecarboxylic acid derivative into a vinylcyclopropaneamino acid derivative; as a result, found that the target compound can be obtained without using Curtius rearrangement which is not suitable for industrial production.

The present invention relates to a method for producing a vinylcyclopropaneamino acid represented by the general formula (6): 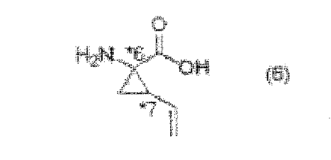 wherein *6 and *7 indicate asymmetric carbon atoms,
comprising the step of reacting a vinylcyclopropaneamidecarboxylic acid derivative represented by the general formula (5): 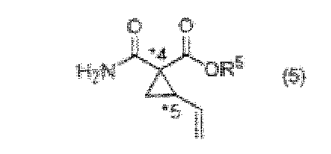 wherein R⁵ is a hydrogen atom, a substituted or unsubstituted alkyl group having 1 to 4 carbon atoms, a substituted or unsubstituted aralkyl group having 7 to 11 carbon atoms, or a metal atom; *4 and *5 indicate asymmetric carbon atoms,
or the salt thereof with a halogenating agent in the presence of a base.

The present invention is also related to a method for producing a vinylcyclopropaneamino acid derivative represented by the general formula (4): 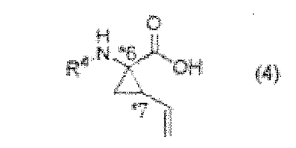 wherein R⁴ is a substituted or unsubstituted alkyloxycarbonyl group having 1 to 15 carbon atoms, a substituted or unsubstituted aralkyloxycarbonyl group having 7 to 12 carbon atoms, a substituted or unsubstituted acyl group having 2 to 12 carbon atoms, or a hydrogen atom; *6 and *7 indicate asymmetric carbon atoms,
comprising the step of protecting the amino group of the vinylcyclopropaneamino acid produced by the above-described method.

### EFFECT OF THE INVENTION

By the present invention method, a vinylcyclopropanecarboxylic acid derivative with high optical purity can be obtained at low cost, and an optically active vinylcyclopropaneamino acid with high optical purity can be obtained at low cost.

### MODE FOR CARRYING OUT THE INVENTION

Hereinafter, the present invention is described in detail. In the present application, a diastereomer salt of an optically active vinylcyclopropanecarboxylic acid derivative and an amine compound is described as a diastereomer salt of optically active vinylcyclopropanecarboxylic acid derivative - amine compound in some cases.

First, the method for producing an optically active vinylcyclopropanecarboxylic acid derivative is described. In the method, the step of reacting a racemic vinylcyclopropanecarboxylic acid derivative represented by the general formula (1): 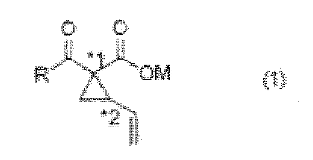 (referred to as "compound (1)" in some cases) with an optically active amine compound, to be a diastereomer salt of optically active vinylcyclopropanecarboxylic acid derivative - amine compound, is contained.

In the formula (1), "M" is a hydrogen atom or a metal atom. As the metal, an alkali metal such as lithium, sodium and potassium; an alkaline earth metal such as magnesium and calcium; and others are exemplified. As the "M", sodium, potassium and a hydrogen atom are preferable, a hydrogen atom is more preferable.

In the case where "M" is a hydrogen atom, in other words, the compound (1) is a carboxylic acid, the compound (1) may be an amine salt. The amine for forming such an amine salt is not particularly limited, but is exemplified by a tertiary amine such as trimethylamine, triethylamine, ethyldiisopropylamine, N,N-dimethylaniline, N,N-diethylaniline, N,N-dimethylaminopyridine, pyridine, picoline, lutidine, N,N,N,N-tetramethyl-1,2-ethylenediamine, N,N,N,N-tetramethyl-1,3-propanediamine and N,N,N,N-tctramethyl-1,6-hexanediamine; a secondary amine such as dimethylamine, diethylamine, dibutylamine, dicyclohexylamine and dibenzylamine; a primary amine such as butylamine, benzylamine and cyclohexylamine.

In the formula (1), R is NH₂, a substituted or unsubstituted alkoxy group having 1 to 4 carbon atoms, a substituted or unsubstituted aryloxy group having 6 to 10 carbon atoms, or a substituted or unsubstituted aralkyloxy group having 7 to 11 carbon atoms.

The substituted or unsubstituted alkoxy group having 1 to 4 carbon atoms is exemplified by a methoxy group, an ethoxy group, a propoxy group, an isopropoxy group and others. The substituted or unsubstituted aryloxy group having 6 to 10 carbon atoms is exemplified by a phenoxy group, a p-methoxyphenoxy group and others. The substituted or unsubstituted aralkyloxy group having 7 to 11 carbon atoms is exemplified by a benzyloxy group, a p-methoxybenzyloxy group and others. The substituent group is exemplified by an alkyl group, an aryl group, an aralkyl group, an amino group, a nitro group, a sulfonyl group, a halogen atom, a hydroxy group, an acyloxy group, an alkoxy group and others.

Among the examples, NH₂, a methoxy group, an ethoxy group and a benzyloxy group are preferable; NH₂ and a methoxy group are more preferable; and NH₂ is particularly preferable.

In the formula (1), the *1 and *2 indicate asymmetric carbon atoms. The *1 and *2 may indicate (R) or (S). The compound (1) has a racemic form; and four optical isomers of (1R,2R), (1R,2S), (1S,2R) and (1S,2S) may be equally contained, the enantiomers of (1R,2R) and (1S,2S) may be excess, and the enantiomers of (1R,2S) and (1S,2R) may be excess. Among the above cases, it is preferable that the enantiomers of (1R,2R) and (1S,2S), which is useful as pharmaceutical intermediates, are excess.

As the production method of the racemic vinylcyclopropanecarboxylic acid derivative represented by (1) of which R is an alkoxy group, an aryloxy group or an aralkyloxy group, the method in which a racemic vinylcyclopropanemalonic acid diester is hydrorized. By the method, the compound can be easily synthesized with high yield.

The compound of which R is NH₂ can be easily synthesized with high yield by, for example, a racemic vinylcyclopropanemalonic acid diester is amidated using ammonia in accordance with Synthetic Communications, 1994, 24, 1477, and then hydrolyzing the ester.

A racemic vinylcyclopropanemalonic acid diester can be easily synthesized with high yield by, for example, reacting a malonic acid diester with 1,4-dihalo-2-butene in the presence of a base in accordance with Journal of Organic Chemistry, 2004, 69, 2427.

The optically active amine compound to be used is exemplified by an optically active 1-arylethylamine derivative represented by the general formula (2): 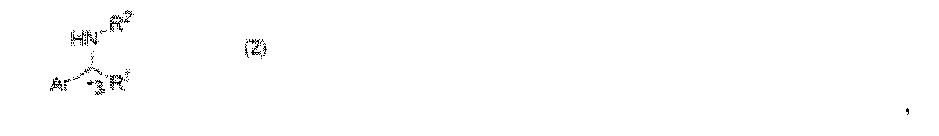 and a naturally-derived optically active amine derivative such as cinchonidine, cinchonine, quinine and quinidine; an amino acid derivative such as lysine, a prolineamide derivative, a proline benzyl ester, alaninol and phenylglycinol; 2-amino-1,2-diphenylethanol, 2-amino-1,2-diphenylamine and others. Among the examples, the optically active 1-arylethylamine derivative represented by the formula (2) is preferable, since both of the (R)-form and (S)-form thereof are industrially available in a large amount.

The R¹ in the formula (2) is an alkyl group having 1 to 3 carbon atoms. The alkyl group having 1 to 3 carbon atoms is exemplified by a methyl group, an ethyl group and a propyl group. Among the examples, a methyl group is preferable, since the compound having the group is available at low cost.

The R² in the formula (2) is a substituted or unsubstituted aralkyl group having 7 to 11 carbon atoms or a hydrogen atom. The substituent is exemplified by those exemplified for R.

The substituted or unsubstituted aralkyl group having 7 to 11 carbon atoms is exemplified by a benzyl group, a p-methoxybenzyl group, an m-methoxybenzyl group, an o-methoxybenzyl group, a 2,3-dimethoxybenzyl group, a p-chlorobenzyl group, an m-chlorobenzyl group, an o-chlorobenzyl group, a 3,4-dichlorobenzyl group, a 3,5-dichlorobenzyl group, a 2,6-dichlorobenzyl group, a p-nitrobenzyl group, an m-nitrobenzyl group, an o-nitrobenzyl group, a p-methylbenzyl group, an m-methylbenzyl group, an o-methylbenzyl group, a 2,3-dimethylbenzyl group, a 2,4-dimethylbenzyl group, a 3,4-dimethylbenzyl group, a 1-naphthylmethyl group, 2-naphthylmethyl group, a2-hydorxybenzyl group and others.

Among the examples, a benzyl group, a p-methylbenzyl group, an m-methylbenzyl group, an o-methylbenzyl group, a 2,3-dimethylbenzyl group, a p-chlorobenzyl group, an m-chlorobenzyl group, an o-chlorobenzyl group, a 3,4-dichlorobenzyl group, a 3,5-dichlorobenzyl group, a p-nitrobenzyl group, an m-nitrobenzyl group, an o-nitrobenzyl group and a hydrogen atom are preferable; and a benzyl group, an o-chlorobenzyl group, a p-chlorobenzyl group, an m-chlorobenzyl group and a hydrogen atom are more preferable.

In the formula (2), the "Ar" is a substituted or unsubstituted aryl group having 6 to 10 carbon atoms. The substituent is exemplified by those exemplified for R.

The substituted or unsubstituted aryl group having 6 to 10 carbon atoms is exemplified by a phenyl group, an o-methylphenyl group, an m-methylphenyl group, a p-methylphenyl group, a 2,3-dimethylphenyl group, an o-methoxyphenyl group, an m-methoxyphenyl group, a p-methoxyphenyl group, a 3,4-dimethoxyphenyl group, an o-chlorophenyl group, an m-chlorophenyl group, a p-chlorophenyl group, a 3,4-dichlorophenyl group, an o-fluorophenyl group, an m-fluorophenyl group, a p-fluorophenyl group, an o-bromophenyl group, an m-bromophenyl group, a p-bromophenyl group, an o-nitrophenyl group, an m-nitrophenyl group, a p-nitrophenyl group, an o-hydroxyphenyl group, an m-hydroxyphenyl group, a p-hydroxyphenyl group, a 1-naphthylgroup, a 2-naphthyl group and others. Among the examples, a phenyl group, a p-methylphenyl group, a p-chlorophenyl group, a p-methoxyphenyl group, a 1-naplathyl group and a 2-naphthyl group are preferable, and a phenyl group, a 1-naphthyl group and a 2-naphthyl group are more preferable.

As the combination of R¹, R² and Ar, the combination that R¹ is a methyl group, and R² is a benzyl group, a p-methylbenzyl group, an m-methylbenzyl group, an o-methylbenzyl group, a 2,3-dimethylbenzyl group, a p-chlorobenzyl group, an m-chlorobenzyl group, an o-chlorobenzyl group, a 3,4-chlorobenzyl group, a 3,5-dichlorobenzyl group, a p-nitrobenzyl group, an m-nitrobenzyl group, an o-nitrobenzyl group or a hydrogen atom, and Ar is a phenyl group, a p-methylphenyl group, a p-chlorophenyl group, a p-methoxyphenyl group, a 1-naphthyl group or a 2-naphthyl group is preferable. [0049]
The combination that R¹ is a methyl group, and R² is a benzyl group, an o-chlorobenzyl group, a p-chlorobenzyl group, an m-chlorobenzyl group or a hydrogen atom, and Ar is a phenyl group, a 1-naphthyl group or a 2-naphthyl group is more preferable.

The following combinations are most preferable:
R¹ is a methyl group, R² is a benzyl group, Ar is a phenyl group;
R¹ is a methyl group, R² is a p-chlorobenzyl group, Ar is a phenyl group;
R¹ is a methyl group, R² is an m-chlorobenzyl group, Ar is a phenyl group;
R¹ is a methyl group, R² is an o-chlorobenzyl group, Ar is a phenyl group;
R¹ is a methyl group, R² is a hydrogen atom, Ar is a phenyl group;
R¹ is a methyl group, R² is a hydrogen atom, Ar is a 1-naphthyl group.

In the formula (2), *3 indicates an asymmetric carbon atom. The *3 may indicates (R) or (S).

The optically active amine compound to be used may be a free amine or an amine salt. Such an amine salt is not limited, and is exemplified by a hydrochloride salt, a sulfate salt, a carbonate salt and others. Among the examples, the amine compound is preferably a free amine or a hydrochloride salt, more preferably a free amine.

The use amount of the optically active amine may be generally not less than 0.1 times by mole, preferably 0.3 to 2.0 times by mole, more preferably 0.3 to 1.5 times by mole, even more preferably 0.4 to 1.5 times by mole, particularly preferably 0.5 to 1.1 times by mole, the most preferably 0.6 to 1.0 times by mole, relative to vinylcyclopropanecarboxylic acid derivative (1).

The solvent used in the reaction is not limited, and exemplified by an aprotic polar solvent such as N,N-dimethylformamide (DMF), dimethylsulfoxide (DMSO), N-methylpyrrolidone and hexamethylphosphatetriamide; a hydrocarbon solvent such as hexamethylbenzene, toluene, n-hexane and cyclohexane; an ether solvent such as diethyl ether, tetrahydrofuran (THF), diisopropyl ether, methyl tert-butyl ether and dimethoxyethane; a halogenated solvent such as chlorobenzene, methylene chloride, chloroform and 1,1,1-trichloroethane; an ester solvent such as ethyl acetate and butyl acetate; a nitrile solvent such as acetonitrile and butyronitrile; an alcohol solvent such as methanol, ethanol, isopropanol, butanol and octanol; water; and others One of the solvents may be used by itself, or plural solvents may be used in combination.

Among the examples, acetone, toluene, methyl tert-butyl ether, ethyl acetate, acetonitrile, methanol, ethanol and isopropanol are preferable, and acetone, toluene, ethyl acetate, acetonitrile and isopropanol are more preferable

The use amount of the solvent is 1 to 200 times by weight, preferable 1 to 50 times by weight, relative to the compound (1).

The reaction temperature is generally within the range of 0 to 120°C, preferably within the range of 10 to 100°C, more preferably 10 to 80°C

The thus obtained diastereomer salt of vinylcyclopropanecarboxylic acid derivative - amine compound is subjected to a crystallization step for optical resolution

The temperature for crystallization is generally within the range of -50 to 70°C, preferably within the range of -20 to 50°C, more preferably -10 to 50°C

The power for stirring per unit volume during crystallization is generally not less than 0.01 kW/m³, preferably not less than 0.1 kW/m³, more preferably not less than 0.2 kW/m³. If the corresponding flowability is obtainable, it is not necessarily needed to use an agitation blade and, for example, the circulation of the reaction mixture may be applicable.

The crystallization method is not limited, and any crystallization method may be used. For example, the following methods may be used:
the method in which a suitable solvent and an optically active amine compound are added to the compound (1), and then the mixture is heated to be homogenous, and then the mixture is cooled for crystallization;
the method in which the compound (1) is dissolved or dispersed in a suitable solvent, and an optically active amine compound is added thereto for crystallization;
the method in which an optically active amine compound is dissolved or dispersed in a suitable solvent, and the compound (1) is added thereto for crystallization.
The procedure such as seed crystal addition can be further combined with the above methods.

Thus obtained crystal of the diastereomer salt of optically active vinylcyclopropanecarboxylic acid derivative - amine compound can be obtained as a wet solid by solid-liquid separation procedure such as centrifugation, filtration under pressure and filtration under reduced pressure, and further washing the cake. Furthermore, dry crystal can be obtained by drying under reduced pressure.

When the obtained crystal is used for the reaction of the next step, dry crystal may be used or wet crystal may be directly used.

The isolated diastereomer salt of optically active vinylcyclopropanecarboxylic acid derivative - amine comopound is converted back to the optically active vinylcyclopropanecarboxylic acid derivative.

As the method to convert the diastereomer salt to the carboxylic acid derivative, for example, extraction procedure using water and an organic solvent in the presence of an acid or a base may be carried out. By extracting the diastereomer salt of optically active vinylcyclopropanecarboxylic acid derivative - amine compound using an organic solvent - water mixture in the presence of an acid, the optically active vinylcyclopropanecarboxylic acid derivative can be separately extracted in the organic layer to be obtained and the optically active amine compound can be separately extracted in the aqueous layer to be obtained. Alternatively extraction procedure is carried out using water and an organic solvent in the presence of a base, so that the optically active vinylcyclopropanecarboxylic acid derivative can be separately extracted in the aqueous layer to be obtained and the optically active amine compound can be separately extracted in the organic layer to be obtained.

The solvent for extraction is not limited, and the solvent exemplified in the above as the reaction solvent can be used. One of the solvents may be used by itself, or plural solvents may be used in combination. Among the examples, a hydrocarbon solvent, an ether solvent, a halogenated solvent and an ester solvent are preferable, and toluene, methyl tert-butyl ether, chlorobenzene, methylene chloride and ethyl acetate are more preferable.

The use amount of the solvent is 1 to 200 times by weight, preferable 1 to 50 times by weight, relative to the diastereomer salt of optically active vinylcyclopropanecarboxylic acid derivative - amine compound.

The use amount of the water is 0.5 to 200 times by weight, preferably 0.5 to 50 times by weight, relative to the diastereomer salt of optically active vinylcyclopropanecarboxylic acid derivative - amine compound.

The acid to be used is not limited, and is exemplified by a mineral acid such as hydrochloric acid, sulfuric acid and phosphoric acid; an organic acid such as methanesulfonic acid, p-toluenesulfonic acid, acetic acid and trifluoroacetic acid. One of the acids may be used by itself, or plural acids may be used in combination. Among the examples, hydrochloric acid and sulfuric acid are preferable.

The use amount of the solvent is not less than the same mole, preferably 0.5 to 200 times by mole, more preferably 0.5 to 50 times by mole, even more preferably 1 to 10 times by mole, especially preferably 1 to 5 times by mole, relative to the diastereomer salt of optically active vinylcyclopropanecarboxylic acid derivative - amine compound.

The base to be used may be an inorganic base or an organic base. Such an inorganic base is exemplified by a metal hydroxide such as sodium hydroxide, potassium hydroxide, lithium hydroxide, magnesium hydroxide and barium hydroxide; a carbonate such as sodium carbonate, potassium hydroxide and sodium hydrogencarbonate; and others.

The organic base is not particularly limited, but a tertiary amine is preferable. Such a tertiary amine is exemplified by a trialkylamine having 1 to 12 carbon atoms, such as trimethylamine, triethylamine and ethyldiisopropylamine; a tertiary amine consisting of an alkyl group having 1 to 4 carbon atoms and a phenyl group, such as N,N-dimethylaniline, N,N-diethylaniline and N,N-dimethylaminopyridine; a nitrogen-containing organic base such as pyridine, picoline and lutidine; an N,N,N,N-tetramethyl-α,ω-alkyldiamine having I to 10 carbon atoms, such as N,N,N,N-tetramethyl-1,2-ethylenediamine, N,N,N,N-tetramethyl-1,3-propanediamine and N,N,N,N-tetramethyl-1,6-hexanediamine.

One of the bases may be used by itself, or plural bases may be used in combination. Among the examples, lithium hydroxide, sodium hydroxide and potassium hydroxide are preferable, since the bases are easily available at low cost.

The use amount of the base is not less than the same mole, preferably 1 to 200 times by mole, more preferably 1 to 50 times by mole, even more preferably 1 to 10 times by mole, especially preferably 1 to 5 times by mole, relative to the diastereomer salt of optically active vinylcyclopropanecarboxylic acid derivative - amine compound.

The temperature for the extraction procedure is generally within the range of -20 to 120°C, preferably -20 to 90°C, more preferably -20 to 70°C.

By the above-mentioned method, the optically active vinylcyclopropanecarboxylic acid derivative with high optical purity can be obtained easier than conventional methods.

The diastereomer salt of optically active vinylcyclopropanecarboxylic acid derivative - amine compound, represented by the general formula (3): 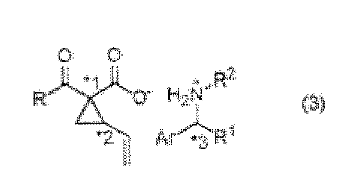 is a novel compound which was found and developed as a pharmaceutical intermediate by the study of the present inventor, and has not been described in any documents.

In the formula (3), R and M are the same as those of the formula (1).

In the formula (3), R¹, R² and Ar are the same as those of the formula (2).

In the formula (3), *1 and *2 indicate asymmetric carbon atoms. The *1 and *2 may indicate (R) or (S). Any one of the isomer of (1R,2R), (1R,2S), (1S,2R) or (1S,2S) is excess in the diastereomer salt of optically active vinylcyclopropanecarboxylic acid derivative-amine compound, since the salt is an optically active. Among the salts, the salt excessively-containing (1S,2S) is preferable.

In the formula (3), *3 indicates an asymmetric carbon atom. The *3 may indicate (R) or (S).

Next, a method comprising the step of reacting a vinylcyclopropaneamidecarboxylic acid derivative represented by the general formula (5): 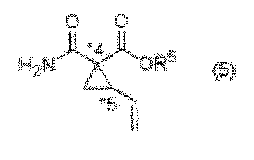 (hereinafter, referred to as "compound (5)") or the salt thereof with a halogenating agent in the presence of a base, to produce a vinylcyclopropaneamino acid (hereinafter, refer as "compound (6)") represented by the general formula (6): 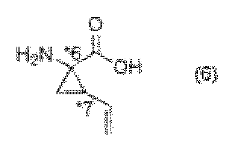 is described.

In the formula (5), R⁵ is a hydrogen atom, a substituted or unsubstituted alkyl group having 1 to 4 carbon atoms, a substituted or unsubstituted aralkyl group having 7 to 11 carbon atoms, or a metal atom. The substituent is exemplified by those of R.

The substituted or unsubstituted alkyl group having 1 to 4 carbon atoms is exemplified by a methyl group, an ethyl group, an isopropyl group and others.

The substituted or unsubstituted aralkyl group having 7 to 11 carbon atoms is exemplified by a benzyl group, a p-methoxybenzyl group and others.

The metal atom is exemplified by an alkali metal such as lithium, sodium and potassium; an alkaline earth metal such as magnesium and calcium; and others.

Among the examples, R⁵ is preferably a hydrogen atom, a methyl group, an ethyl group, sodium and potassium, and particularly preferably a hydrogen atom.

In the case where R⁵ is a hydrogen atom, in other words, the compound (5) is a carboxylic acid, the compound (5) may be an amine salt. The amine for forming such an amine salt is not particularly limited, but is exemplified by a tertiary amine such as trimethylamine, triethylamine, ethyldiisopropylamine, N,N-dimethylaniline, N,N-diethylaniline, N,N-dimethylaminopyridine, pyridine, picoline, lutidine, N,N,N,N-tetramethyl-1,2-ethylenediamine, N,N,N,N-tetramethyl-1,3-propanediamine and N,N,N,N-tetramethyl-1,6-hexanediamine; a secondary amine such as dimethylamine, diethylamine, dibutylamine, dicyclohexylamine and dibenzylamine; a primary amine such as butylamine, benzylamine and cyclohexylamine.

The amine may be an optically active amine, and is exemplified the optically active 1-arylethylamine derivative represented by the formula (2); a naturally-derived optically active amine derivative such as cinchonidine, cinchonine, quinine and quinidine; an amino acid derivative such as lysine, a prolineamide derivative, a proline benzyl ester, alaninol and phenylglycinol; 2-amino-1,2-diphenylethanol, 2-amino-1,2-diphenylamine and others. Among the examples, the amine salt with the optically active 1-arylethylamine derivative represented by the formula (2) is preferable.

In the formula (5), *4 and *5 indicate asymmetric carbon atoms. The *4 and *5 may indicate (R) or (S).

The compound (5) to be used may be racemic or optically active, preferably optically active. The optically active form is not limited and any one excesively-having (1R,2R), (1R,2S), (1S,2R) or (1S,2S) may be used; but among the examples, the compound (5) preferably contains excess (1S,2S).

When the optically active from is used, the diastereomer salt of optically active vinylcyclopropanecarboxylic acid derivative - amine compound produced by the above-mentioned method may be used, or the free optically active cyclopropaneamidecarboxylic acid derivative obtained by releasing procedure may be used. Alternatively, the active form obtained by the other methods may be used.

In the formula (6), *6 and *7 indicate asymmetric carbon atoms. The *6 and *7 may indicate (R) or (S).

The reaction proceeds while the configuration is maintained. The position at 1 in the configuration of the produced compound (6) is opposite to the vinylcyclopropaneamidecarboxylic acid derivative (5) in accordance with the CIP priority rule. In other words, the compound (6) of (1S,2R) is produced from the compound (5) of (1R,2R), the compound (6) of (1S,2S) is produced from the compound (5) of (1R,2S), the compound (6) of (1R,2R) is produced from the compound (5) of (1S,2R), the compound (6) of (1R,2S) is produced from the compound (5) of (1S,2S). The configuration of the compound (6) is preferably (1R,2S).

The base to be used may be an inorganic base or an organic base. Such an inorganic base is exemplified by a metal hydroxide such as sodium hydroxide, potassium hydroxide, lithium hydroxide, magnesium hydroxide and barium hydroxide; a carbonate such as sodium carbonate, potassium hydroxide and sodium hydrogencarbonate; and others.

The organic base is not particularly limited, but a tertiary amine is preferable. Such a tertiary amine is exemplified by a trialkylamine having 1 to 12 carbon atoms, such as trimethylamine, triethylamine and ethyldiisopropylamine; a tertiary amine consisting of an alkyl group having 1 to 4 carbon atoms and a phenyl group, such as N,N-dimethylaniline, N,N-diethylaniline and N,N-dimethylaminopyridine; a nitrogen-containing organic base such as pyridine, picoline and lutidine; an N,N,N,N-tetramethyl-α,ω-alkyldiamine having 1 to 10 carbon atoms, such as N,N,N,N-tetramethyl-1,2-ethylenediamine, N,N,N,N-tetramethyl-1,3-propanediamine and N,N,N,N-tetramethyl-1,6-hexanediamme; and others.

One of the bases may be used by itself, or plural bases may be used in combination. Among the examples, lithium hydroxide, sodium hydroxide and potassium hydroxide are preferable, since the bases are easily available at low cost.

The use amount of the base is not less than the same mole, preferably I to 20 times by mole, more preferably 1 to 10 times by mole, relative to the compound (5).

The base to be used in the reaction may be directly used without or with being diluted with water or an organic solvent.

The halogenating agent to be used in the reaction is exemplified by sodium hypochlorite, potassium hypochlorite, sodium hypobromite, chlorine, bromine, iodine, N-chlorosuccinimide (NCS), N-bromosuccinimide (NBS), N-iodosuccinimide (NIS), N-chloroisocyanuric acid and others. Among the examples, sodium hypochlorite and sodium hypobromite are preferably, and sodium hypochlorite is more preferable.

The halogenating agent may be directly used, or the halogenating agent diluted with water or an organic solvent may be used. In particular, when sodium hypochlorite is used, the solution thereof is generally used.

The halogenating agent may be prepared in the reaction mixture. For example, the methanol solution of sodium hypobromite can be prepared by reacting bromine with sodium methoxide in methanol.

The use amount of the halogenating agent may be not less than 1 time by mole, preferably 1 to 20 times by mole, more preferably 1 to 10 times by mole, relative to the compound (5).

The use amount of water may be 1 to 200 times by weight, preferably 1 to 50 times by weight, relative to the compound (5).

An organic solvent is preferably used in the reaction, since the reaction is accelerated in some cases.

The solvent to be used is not limited, and the solvent exemplified as the reaction solvent in the description of the reaction of the compound (1) and optically active amine compound may be used. One of the solvents may be used by itself, or plural solvents may be used in combination. Among the examples, an ether solvent, an ester solvent, an alcohol solvent and water are preferable, tetrahydrofuran (THF), ethyl acetate, methanol, ethanol, isopropanol, n-propanol and water are more preferable.

The reaction temperature is generally within the range of -30 to 120°C, preferably within the range of -20 to 100°C, more preferably -20 to 80°C.

After the reaction, a general work-up process may be carried out for obtaining a crude product from the reaction mixture. For example, an extraction procedure is carried out using a general extraction solvent such as ethyl acetate, diethyl ether, methylene chloride, toluene and hexane. Then the reaction solvent and extraction solvent are distilled away by a procedure such as heating under reduced pressure from the obtained extract, to obtain the target compound. Alternatively an acid such as hydrochloric acid and sulfuric acid is added to the reaction mixture and the pH of the reaction mixture is adjusted to the isoelectric point of the amino acid for crystallization, and then the product is obtained by filtration. Furthermore, the reaction solvent is distilled away by a procedure such as heating under reduced pressure or the solvent is substituted by the other solvent, and then the above procedure may be carried out.

Thus obtained target compound is almost pure, but the purity thereof may be further improved by a general purification method such as crystallization, fractional distillation and column chromatography.

As necessary, thus obtained compound (6) may be derived to the vinylcyclopropaneamino acid derivative represented by the general formula (4): 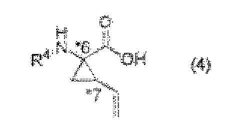 (hereinafter, referred to as "compound (4)").

In the formula (4), R⁴ is a substituted or unsubstituted alkyloxycarbonyl group having 1 to 15 carbon atoms, a substituted or unsubstituted aralkyloxycarbonyl group having 7 to 12 carbon atoms, or a substituted or unsubstituted acyl group having 2 to 12 carbon atoms. The substituent is exemplified by those exemplified for R.

The substituted or unsubstituted alkyloxycarbonyl group having 1 to 15 carbon atoms is exemplified by a t-butyloxycarbonyl group (Boc group), a methoxycarbonyl group (Moc group), a 9-fluorenylmethoxycarbonyl group (Fmoc group) and others. The substituted or unsubstituted aralkyloxycarbonyl group having 7 to 12 carbon atoms is exemplified by a benzyloxycarbonyl group (Cbz group), a p-methoxybenzyloxycarbonyl group and others. The substituted or unsubstituted acyl group having 2 to 12 carbon atoms is exemplified by an acetyl group, a benzoyl group and others. Among the examples, a t-butyloxycarbonyl group (Boc group), a methoxycarbonyl group (Moc group), a 9-fluorenylmethoxycarbonyl group (Fmoc group), a benzyloxycarbonyl group (Cbz group) and an acetyl group are preferable, a t-butyloxycarbonyl group (Boc group) and a benzyloxycarbonyl group (Cbz group) are more preferable.

In the formula (4), *6 and *7 are the same as the above.

In the method for deriving the compound (4) from the compound (6), a general protecting condition may be used. For example, the method can be carried out in accordance with the method described in Theodora W. Greene, Peter G. M. Wuts, Protective Groups in Organic Chemistry (the third edition) JOHN WILEY & SONS, INC.

For example, di-t-butyl dicarbonate is reacted in an appropriate solvent in the presence of a base so that the protection by a t-butyloxycarbonyl (BOC) can be carried out. In addition, benzyloxycarbonyl chloride is reacted in an appropriate solvent in the presence of a base so that the protection by a benzyloxycarbonyl (Cbz) can be carried out.

The compound (6) isolated by the above-mentioned method may be derived to the compound (4); alternatively, the compound (6) may be derived to the compound (4) directly-using the reaction mixture containing the compound (6) without carrying out the isolation procedure.

### EXAMPLES

The present invention is hereinafter explained in more detail with examples; however, the present invention is not limited by the examples.

### Production Example 1: Production method of cis-2-vinyl-1-methoxycarbonylcyclopropanecarboxylic acid

To the methanol solution (150 mL) of trans-1,4-dibromo-2-butene (25.00 g, 0.144 mol) and dimethyl malonate (15.06 g, 0.144 mol), 28wt% methanol solution of sodium methoxide was added dropwise for 30 minutes while the inner temperature was kept at 1 to 5°C. After the dropwise addition, the mixture was stirred at the inner temperature of 24°C for 5 hours. Then the reaction mixture was cooled. While the inner temperature was kept at 1 to 5°C, 14% potassium hydroxide aqueous solution (36.02 g, 0.091 mol) was added dropwise thereto for 30 minutes. After the dropwise addition, the mixture was stirred at the inner temperature of 24°C for 21 hours. Then the mixture was concentrated using a rotary evaporator until the whole amount became about 70 g. After water (45 mL) and t-butyl methyl ether (75 mL) were added to the concentrate, concentrated hydrochloric acid was added thereto until the pH of the mixture became 2.5. The organic layer was obtained by extraction procedure. The organic layer was dried with magnesium sulfate, and then concentrated using a rotary evaporator, to obtain the target compound as a crude product (crude yield: 19.81 g, 92.5%). The ratio of cis form and trans form was 83 : 17.

### Production Example 2: Production method of trans-2-vinyl-1-carbamoylcyclopropanecarboxylic acid (the mixture of (1S,2S)- and (1R,2R)-carbamoylcyclopropanecarboxylic acid at the ratio of 1 : 1}

To the methanol solution (150 mL) of trans-1,4-dibromo-2-butene (25.00 g, 0.144 mol) and dimethyl malonate (15.06 g, 0.144 mol), 28wt% methanol solution of sodium methoxide was added dropwise for 30 minutes while the inner temperature was kept at 1 to 5°C. After the dropwise addition, the mixture was stirred at the inner temperature of 24°C for 5 hours. Then while the inner temperature was kept at 15 to 25°C, ammonia gas (about 15 g, 0.88 mol) was introduced into the reaction mixture for 1 hour. The mixture was stirred at the inner temperature of 25°C for 15 hours. Then the mixture was concentrated using a rotary evaporator until the whole amount became about 142 g. After water (17.44 g) and 30wt% sodium hydroxide aqueous solution (12.17 g, 0.09 mmol) were added to the concentrate, the mixture was stirred at the inner temperature of 40°C for 4 hours. The mixture was concentrated using a rotary evaporator until the whole amount became about 77 g. Water (105 g) and toluene (30 mL) were added thereto, and the aqueous layer was obtained by extraction procedure. Concentrated hydrochloric acid was added thereto until the pH of the mixture became 3.0. The precipitated solid was obtained by filtration, and dried, to obtain the target compound (yield: 12.79 g, 70%). The ratio of cis form and trans form was 2 : 98.

### Example 1: Production method of (1S1,2S)-2-vinyl-1-carbamoylcyclopropanecarboxylate (S)-N-benzyl-1-phenylethylamine salt

The mixture of (1S,2S)- and (1R,2R)-2-vinyl-1-carbamoylcyclopropanecarboxylic acid at the ratio of 1 : 1 produced in Production Example 2 (500 mg, 3.22 mmol) was dispersed in acetonitrile (5 mL), and (S)-N-benzyl-1-phenylethylamine (681 mg, 3.22 mmol) was added thereto at room temperature. The mixture was stirred at the same temperature for 1 hour, and the precipitated crystals were separated by filtration procedure. The crystals were washed with acetonitrile cooled at 0°C (1 mL), and dried, to obtain the target compound (yield: 416.9 mg, 35.3%).

The optical purity of the obtained compound was measured with HPLC; as a result, the optical purity was 95.6%ee.

### HPLC analysis condition

Column: CHIRALCEL OJ-H
Eluent: hexane/isopropanol/trifluoroacetic acid = 90/10/0.1
Flow speed: 0.5 mL/min
Detector: UV 210 nm
Column oven temperature: 30°C
Retention time: (1S,2S)-form: 18.6 min, (1R,2R)-form: 20.4 min
¹H NMR (400 MHz, CDCl₃ / ppm): δ1.52(d, 3H), 1.79(dd, 1H), 1.92(dd, 1H), 2.43(ddd, 1H), 3.74(dd, 2H), 3.98(q, 1H), 5.07(dd, 1H), 5.29(dd, 1H), 5.80-5.89(m, 1H), 6.04(br, 2H), 6.20(br, 1H), 7.24-7.41(m, 10H), 9.21(br, 1H)

### Example 2: Production method of (1S,2S)-2-vinyl-1-carbamoyloyclopropanecarboxylate (S)-N-(4-chlorobenzyl)-1-phenylethylamine salt

The mixture of (1S,2S)- and (1R,2R)-2-vinyl-1-carbamoylcyclopropanecarboxylic acid at the ratio of 1 : 1 synthesized in Production Example 2 (1000 mg, 6.45 mmol) was dispersed in acetonitrile (10 mL), and (S)-N-(4-chlorobenzyl)-1-phenylethylamine (1585 mg, 6.45 mmol) was added thereto at room temperature. The mixture was stirred at the same temperature for 1 hour, and the precipitated crystals were obtained by filtration. The crystals were washed with acetonitrile (2 mL) cooled at 0°C and dried, to obtain the target compound (yield: 788.0 mg, 30.5%).

The optical purity of the obtained compound was measured with HPLC; as a result, the optical purity was 96.6%ee.

### HPLC analysis condition

Column: CHIRALCEL OJ-H
Eluent: hexane/isopropanol/trifluoroacetic acid = 90/10/0.1
Flow speed: 0.5 mL/min
Detector: UV 210 nm
Column oven temperature: 30°C
Retention time: (1S,2S)-form: 18.6 min, (1R,2R)-form: 20.4 min
¹H NMR (400 MHz, CDCl₃ / ppm): δ1.53(d, 3H), 1.80(dd, 1H), 1.92(dd, 1H), 2.41(ddd, 1H), 3.71(dd, 2H), 3.97(q, 1H), 5.09(dd, 1H), 5.31(dd, 1H), 5.80-5.89(m, 1H), 6.51(br,1H), 6.66(br, 2H), 7.19-7.41(m, 9H), 9.28(br, 1H)

### Example 3: Production method of (1S,2S)-2-vinyl-1-carbamoylcyclopropanecarboxylate (S)-N-(3,4-dichlorophenyl)-1-phenylethylamine salt

The mixture of (1S,2S)- and (1R,2R)-2-vinyl-1-carbamoylcyclopropanecarboxylic acid at the ratio of 1 : 1 synthesized in Production Example (200 mg, 1.29 mmol) was dispersed in acetonitrile (2 mL), and (S)-N-(3,4-dichlorophenyl)-1-phenylethylamine (361.2 mg, 1.29 mmol) was added thereto at room temperature. After the addition, the mixture was stirred at the same temperature for 1 hour, and the precipitated crystals were obtained by filtration. The crystals were washed with acetonitrile (1 mL) cooled at 0°C, and dried, to obtain the target compound (yield: 131.4 g, 23.5%).

The optical purity of the obtained compound was measured with HPLC; as a result, the optical purity was 97.9%ee.
¹H NMR (400 MHz, CDCl₃ / ppm): δ1.51(d, 3H), 1.84(dd, 1H), 1.96(dd, 1H), 2.45(ddd, 1H), 3.68(dd, 2H), 3.94(q, 1H), 5.11(dd, 1H), 5.32(dd, 1H), 5.80-5.89(m, 1H), 6.58(br, 1H), 7.09(dd, 1H), 7.26-7.52(m, 8H), 9.11(br, 1H)

### Example 4: Production method of (1S,2S)-2-vinyl-1-carbamoylcyclopropanecarboxylic acid

The (1S,2S)-2-vinyl-1-carbamoylcyclopropanecarboxylate (S)-N-benzyl-1-phenylethylamine salt obtained in Example 1 (250 mg, 682 mmol) was dispersed in toluene (2.5 mL) and water (2.5 mL), and 30wt% sodium hydroxide aqueous solution (363.7 mg) was added to the stirred mixture. After still standing, the aqueous layer was obtained by separating procedure.

### Example 5: Production method of (1R,2S)-2-vinyl-1-(t-butoxycarbonylamino)cyclopropanecarboxylic acid

To the aqueous solution containing sodium (1S,2S)-2-vinyl-1-carbamoylcyclopropanecarboxylate obtained in Example 4, tetrahydrofuran (1 mL) was added. Then 11.7wt% hypochlorous acid aqueous solution (957.1 mg, 1.50 mmol) was added to the mixture at 0°C of a bath. The mixture was stirred at the same temperature for 1 hour, and then stirred at 60°C of a bath for 2 hours. After the reaction mixture was cooled to 26°C, dicarbonic acid di-tert-butyl ester (223.3 mg, 1.02 mmol) was added thereto. After the mixture was stirred at the same temperature for 3 hours, 1M hydrochloric acid was added until the pH of the mixture became 3. Ethyl acetate (20 mL) was added thereto, and the organic layer was obtained by extration. Then the organic layer was washed with water (5 mL). The organic layer was concentrated using a rotary evaporator, to obtain the target compound (yield: 109 mg, 70.0%).

The optical purity of the obtained compound was measured with HPLC; as a result, the optical purity was 97.9%ee.

### HPLC analysis condition

Column: CHIRALCEL OD-H
Eluent: hexane/ethanol = 992/8
Flow speed: 0.75 mL/min
Detector: UV 210 nm
Column oven temperature: 30°C
Retention time: (1S,2S)-form: 19.5 min, (1R,2R)-form: 22.3 min
¹H NMR (400 MHz, DMSO-d₆ / ppm): δ1.24(d, 1H), 1.37(s, 9H), 1.52(d, 1H), 2.06(ddd, 1H), 5.04(dd, 1H), 5.23(dd, 1H), 5.63-5.72(m, 1H)

### Example 6: Production method of cis-2-vinyl-1-methoxycarbonylcyclopropanecarboxylate (R)-1 -(1-naphthyl)ethylamine salt

The mixture of the racemic 2-vinyl-1-methoxycarbonylcyclopropanecarboxylic acid synthesized in accordance with Production Example 1 (cis : trans = 83 : 17, 250 mg, 1.47 mmol) was dispersed in ethyl acetate (2.50 g), and (R)-1-(1-naphthyl)ethylamine (503.2 mg, 1.47 mmol) was added thereto at room temperature. After the addition, the reaction mixture was homogenous; however, after the mixture was stirred for 1 hour at the room temperature, crystals were observed. The mixture was stirred at 50°C of a bath for 20 minutes, and then was cooled until the inner temperature became 30°C by still standing. The crystals were obtained by filtration. The crystals were washed with ethyl acetate (1 mL), and dried, to obtain the target compound (yield: 241.5 mg, 24.1 %).

The optical purity of the obtained compound was measured with HPLC; as a result, the optical purity was 75.4%ee.

### HPLC analysis condition

Column: CHIRALCEL OF
Eluent: hexane/isopropanol/trifluoroacetic acid = 90/10/0.1
Flow speed: 0.6 mL/min
Detector: UV 210 nm
Column oven temperature: 30°C
Retention time: 12.6 min, 18.7 min
¹H NMR (400 MHz, DMSO-d₆ / ppm): δ1.18(dd, 1H), 1.31(dd, 1H), 1.67(d, 3H), 2.13(ddd, 1H), 3.45(s, 1H), 4.89-4.98(m, 2H), 5.13-5.25(m, 2H), 6.70(br, 2H), 7.43-7.57(m, 3H), 7.68(d, 1H), 7.78(d, 1H), 7.85-7.88(m, 1H), 8.02(d, 1H)

### Example 7: Production method of cis-2-vinyl-1-methoxycarbonylcyclopropanecarboxylic acid

The cis-2-vinyl-1-methoxycarbonylcyclopropanecarboxylate (R)-1-(1-naphthyl)ethylamine salt obtained in Example 6 (200 mg, 0.59 mmol) was dispersed in toluene (2.5 mL) and water (2.5 mL), and 35wt% sodium hydroxide aqueous solution (241.1 mg) was added to the stirred dispersion. After still standing, the organic layer was obtained by separating procedure and concentrated. After an azeotropic dehydration procedure using toluene (5 mL) was repeated two times, the concentrate was diluted with toluene so that the whole amount became 2.5 g.

### Example 8: Production method of methyl cis-2-vinyl-1-carbamoylcyclopropanecarboxylate

To the toluene solution containing cis-2-vinyl-1-methoxycarbonylcyclopropanecarboxylic acid obtained in Example 7, triethylamine (89.6 mg, 0.89 mmol) was added and then pivaloyl chloride (74.7 mg, 0.62 mmol) was added at 0°C of a bath. After the mixture was stirred at the same temperature for 1 hour, 28wt% aqueous ammonia (143.5 mg, 2.36 mmol) was added thereto. The mixture was further stirred at 24°C of a bath for 2 hours. Water (2.0 mL) was added to the reaction mixture, and the organic layer was obtained by extraction. The organic layer was dried with anhydrous sodium sulfate, and then concentrated using a rotary evaporator, to obtain the target compound (yield: 55 mg, 50.0%).
¹H NMR (400 MHz, DMSO-d₆ / ppm): δ1.91(dd, 1H), 2.07(dd, 1H), 2.58(ddd, 1H), 3.74(s, 3H), 5.18(dd, 1H), 5.35(dd, 1H), 5.57-5.78(m, 1H), 5.77(br, 2H), 8.22(br, 1H)

### Example 9: Production method of (1S,2S)-2-vinyl-1-carbamoylcyclopropanecarboxylate (S)-N-(2-chlorobenzyl)-1-phenylethylamine salt

The mixture of (1S,2S)- and (1R,2R)-2-vinyl-1-carbamoylcyclopropanecarboxylic acid at the ratio of 1 : 1 obtained in accordance with Production Example 2 (3000 mg, 19.34 mmol) was dispersed in ethyl acetate (21 g), and (S)-N-(2-chlorobenzyl)-1-phenylethylamine (3802 mg, 15.47 mmol) was added thereto at room temperature. The mixture was stirred at the same temperature for 3 hours, and the precipitated crystals were obtained by filtration. The crystals were washed with ethyl acetate (10 mL) and then dried, to obtain the target compound (yield: 3194.8 mg, 41.2%).

The optical purity of the obtained compound was measured with HPLC; as a result, the optical purity was 96.8%ee.
¹H NMR (400 MHz, CDCl₃ / ppm): δ1.56(d 3H), 1.80(dd, 1H), 1.93(dd, 1H), 2.46(ddd, 1H), 3.87(s, 2H), 4.03(q, 1H), 5.09(dd, 1H), 5.30(dd, 1H), 5.75(br, 2H), 5.76-5,85(m, 2H), 6.32(br, 1H), 7.18-7.26(m, 2H), 7.31-7.41(m, 7H), 9.02(br, 1H)

### Example 10: Production method of (1S,2S)-2-vinyl-1-carbamoylcyclopropanecarboxylate (S)-N-(2-chlorobenzyl)-1-phenylethylamine salt

The mixture of (1S,2S)- and (1R,2R)-2-vinyl-1-carbamoylcyclopropanecarboxylic acid at the ratio of 1 : 1 synthesized in accordance with Production Example 2 (2000 mg, 12.89 mmol) was dispersed in ethyl acetate (21 g), and (S)-N-(2-chlorobenzyl)-1-phenylethylamine (2220 mg, 9.02 mmol) was added thereto at 50°C of a bath. The mixture was stirred at the same temperature for 1 hour and then stirred at 15°C of a bath for 17 hours. The precipitated crystals were obtained by filtration. The crystals were washed with ethyl acetate (7.5 mL) and then dried, to obtain the target compound (yield: 2000 mg, 38.7%).

The optical purity of the obtained compound was measured with HPLC; as a result, the optical purity was 98.5%ee.

### Example 11: Production method of (1S,2S)-2-vinyl-1-carbamoylcyclopropanecarboxylate (S)-N-(2-chlorobenzyl)-1-phenylethylamine salt

The mixture of (1S,2S)- and (1R,2R)-2-vinyl-1-carbamoylcyclopropanecarboxylic acid at the ratio of 1 : 1 synthesized in accordance with Production Example 2 (2000 mg, 12.89 mmol) was dispersed in ethyl acetate (21 g), and (S)-N-(2-chlorobenzyl)-1-phenylethylamine (1580 mg, 6.45 mmol) was added thereto at 20°C of a bath. The mixture was stirred at the same temperature for 1. hour, and then further stirred at 20°C of a bath for 17 hours. The precipitated crystals were obtained by filtration. The crystals were washed with ethyl acetate (7.5 mL) and then dried, to obtain the target compound (yield: 2584 mg, 50%).

The optical purity of the obtained compound was measured with HPLC; as a result, the optical purity was 58.0%ee.

### Example 12: Production method of (1S,2S)-2-vinyl-1-carbamoylcyclopropanecarboxylate (S)-N-(2-chlorobenzyl)-1-phenylethylamine salt

The mixture of (1S,2S)- and (1R,2R)-2-vinyl-1-carbamoylcyclopropanecarboxylic acid at the ratio of 1 : 1 synthesized in accordance with Production Example 2 (3000 mg, 19.34 mmol) was dispersed in acetonitrile (21 g), and (S)-N-(2-chlorobenzyl)-1-phenylethylamine (3.800 mg, 15.47 mmol) was added thereto at 60°C of a bath. The mixture was stirred at the same temperature for 1 hour, and further stirred at 30°C of a bath for 2 hours. The precipitated crystals were obtained by filtration. The crystals were washed with acetonitrile (10 mL) and then dried, to obtain the target compound (yield: 3470 mg, 44.8%).

The optical purity of the obtained compound was measured with HPLC; as a result, the optical purity was 89.8%ee.

### Example 13: Production method of (1S,2S)-2-vinyl-1-carbamoylcyclopropanecarboxylate (S)-N-(2-chlorobenzyl)-1-phenylethylamine salt

The mixture of (1S,2S)- and (1R,2R)-2-vinyl-1-carbamoylcyclopropanecarboxylic acid at the ratio of 1: 1 synthesized in accordance with Production Example 2 (3426.2 mg, 22.08 mmol) was dispersed in acetonitrile (31 g), and (S)-N-(2-chlorobenzyl)-1-phenylethylamine (3.800 mg, 15.47 mmol) was added thereto at 70°C of a bath. The mixture was stirred at the same temperature for 1 hour, and then further stirred at 40°C of a bath at 2 hours. The precipitated crystals were obtained by filtration. The crystals were washed with acetonitrile (10 mL) and then dried, to obtain the target compound (yield: 3322 mg, 37.5%).

The optical purity of the obtained compound was measured with HPLC; as a result, the optical purity was 96.3%ee.

### Example 14: Production method of (1S,2S)-2-vinyl-1-carbamoylcyclopropanecarboxylate (S)-N-(2-chlorobenzyl)-1-phenylethylamine salt

The mixture of (1S,2S)- and (1R,2R)-2-vinyl-1-carbamoylcyclopropanecarboxylic acid at the ratio of 1: 1 synthesized in accordance with Production Example 2 (3000 mg, 19.34 mmol) was dispersed in acetone (21 g), and (S)-N-(2-chlorobenzyl)-1-phenylethylamine (3.800 mg, 15.47 mmol) was added thereto at 50°C of a bath. The mixture was stirred at the same temperature for 1 hour, and further stirred at 20°C of a bath for 2 hours. The precipitated crystals were obtained. The crystals were washed with acetone (10 mL) and then dried, to obtain the target compound (yield: 3017 mg, 38.9%).

The optical purity of the obtained compound was measured with HPLC; as a result, the optical purity was 95.7%ee.

### Example 15: Production method of (1S,2S)-2-vinyl-1-carbamoylcyclopropanecarboxylate (S)-N-(4-chlorobenzyl)-1-phenylethylamine salt

The mixture of (1S,2S)- and (1R,2R)-2-vinyl-1-carbamoylcyclopropancarboxylic acid at the ratio of 1:1 synthesized in accordance with Production Example 2 (1000 mg, 6.44 mmol) was dispersed in toluene (6.4 g), and (S)-N-(4-chlorobenzyl)-1-phenylethylamine (1519 mg, 5.16 mmol) was added thereto at 50°C of a bath. The mixture was stirred at the same temperature for 1 hour, and further stirred at 0°C of a bath for 20 hours. The precipitated crystals were obtained by filtration. The crystals were washed with acetonitrile (2 mL) and then dried, to obtain the target compound (yield: 503 mg, 19.5%).

The optical purity of the obtained compound was measured with HPLC; as a result, the optical purity was 97.2%ee.

### Example 16: Production method of (1S,2S)-2-vinyl-1-carbamoylcyclopropanecarboxylate (S)-N-(4-methylbenzyl)-1-phenylethylamine salt

The mixture of (1S,2S)- and (1R,2R)-2-vinyl-1-carbamoylcyclopropanecarboxylic acid at the ratio of 1:1 synthesized in accordance with Production Example 2 (1000 mg, 6.44 mmol) was dispersed in acetonitrile (7.0 g), and (S)-N-(4-methylbenzyl)-1-phenylethylamine (1161 mg, 5.15 mmol) was added thereto at 30°C of a bath. The mixture was stirred at the same temperature for 15 hours, and then further stirred at 0°C of a bath for 7 hours. The precipitated crystals were obtained. The crystals were washed with acetonitrile (2 mL) and then dried, to obtain the target compound (yield: 287 mg, 11.7%).

The optical purity of the obtained compound was measured with HPLC; as a result, the optical purity was 97.6%ee.

## Claims

1. A method for producing an optically active vinylcyclopropanecarboxylic acid derivative, comprising the step of reacting a racemic vinylcyclopropanecarboxylic acid derivative represented by the general formula (1): 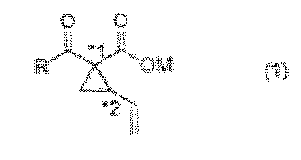 wherein, R is NH₂, a substituted or unsubstituted alkoxy group having to 4 carbon atoms, a substituted or unsubstituted aryloxy group having 6 to 10 carbon atoms, or a substituted or unsubstituted aralkyloxy group having 7 to 11 carbon atoms; M is a hydrogen atom or a metal atom; * 1 and *2 indicate asymmetric carbon atoms,
with an optically active amine compound, to obtain a diastereomer salt of optimally active vinylcyclopropanecarboxylic acid derivative - amine compound.

2. The production method according to claim 1, wherein the optically active amine compound is an optically active 1-arylethylamine derivative represented by the general formula (2): 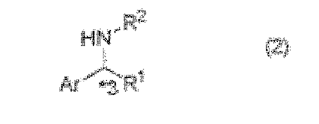 wherein, R¹ is an alkyl group having 1 to 3 carbon atoms; R² is a substituted or unsubstituted aralkyl group having 7 to 11 carbon atoms, or a hydrogen atom; Ar is a substituted or unsubstituted aryl group having 6 to 10 carbon atoms; *3 indicates an asymmetric carbon atom.

3. The production method according to claim 2, wherein R¹ is a methyl group.

4. The production method according to claim 2 or 3, wherein Ar is a phenyl group, a 1 - naphthyl group or a 2-naphthyl group.

5. The production method according to any one of claims 1 to 4, wherein R is NH₂.

6. A diastereomer salt of optically active vinylcyclopropanecarboxylic acid derivative - amine compound, represented by the general formula (3): 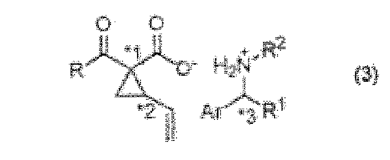 wherein R, R¹, R², Ar, *1, *2 and *3 are the same as the above.

7. The salt according to claim 6, wherein R¹ is a methyl group.

8. The salt according to claim 6 or 7, wherein Ar is a phenyl group, a 1 -naphthyl group or a 2-naphthyl group.

9. The salt according to any one of claims 6 to 8, wherein R is NH₂.

10. A method for producing a vinylcyclopropaneamino acid represented by the general formula (6): 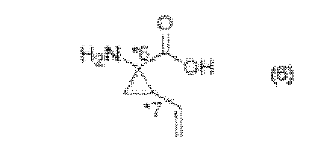 wherein *6 and *7 indicate asymmetric carbon atoms,
comprising the step of reacting a vinylcycloprapaneamidecarboxylic acid derivative represented by the general formula (5): 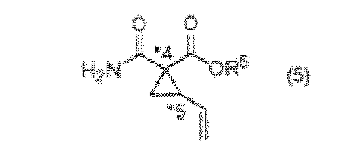 wherein R⁵ is a hydrogen atom, a substituted or unsubstituted alkyl group having 1 to 4 carbon atoms, a substituted or unsubstituted aralkyl group having 7 to 11 carbon atoms, or a metal atom; *4 and *5 indicate asymmetric carbon atoms,
or the salt thereof with a halogenating agent in the presence of a base.

11. A method for producing a vinylcyclopropaneamino acid derivative represented by the general formula (4): 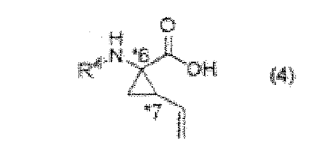 wherein R⁴ is a substituted or unsubstituted alkyloxycarbonyl group having 1 to 15 carbon atoms, a substituted or unsubstituted aralkyloxycarbonyl group having 7 to 12 carbon atoms, a substituted or unsubstituted acyl group having 2 to 12 carbon atoms, or a hydrogen atom; *6 and *7 are the same as the above,
comprising the step of protecting the amino group of the vinylcyclopropaneamino acid produced by the method according to claim 10.

12. The production method according to claim 10 or 11, wherein the vinylcyclopropaneamidecarboxylic acid derivative is the optically active vinylcyclopropaneamidecarboxylic acid derivative or the diastereomer salt of optically active vinylcyclopropanecarboxylic acid derivative - amine compound produced by the production method according to any one of claims 1 to 5.
